# EUROPEAN PATENT SPECIFICATION

(11) **EP 1 735 625 B1**
(45) Date of publication and mention of the grant of the patent: **04.11.2009**
(21) Application number: 05718619.9
(22) Date of filing: 01.04.2005
(51) Int. Cl.: G01N 33/68, G01N 33/92, A61K 31/00, G01N 33/573

(54) **SCREENING FOR A MODULATOR OF ATHEROGENIC LIPOPROTEIN RETENTION**
SCREENINGVERFAHREN FÜR MODULATOREN DER ATHEROGENEN LIPOPROTEINEN RETENTION
PROCÉDÉ DE CRIBLAGE D'UN MODULATEUR DE LA RÉTENTION DES LIPOPROTÉINES ATHEROGÈNES

(30) Priority: 02.04.2004 DK 200400538
(43) Date of publication of application: 27.12.2006
(73) Proprietor: Bjursell, Gunnar, 433 31 Partille (SE); Nilsson, Jeanette Annika, 42737 Billdal (SE); Ellmark, Sara, 427 40 Billdal (SE)
(72) Inventor: Bjursell, Gunnar, 433 31 Partille (SE); Nilsson, Jeanette Annika, 42737 Billdal (SE); Ellmark, Sara, 427 40 Billdal (SE)
(74) Representative: Wright, Andrew John
(86) International application number: PCT/IB2005/051095
(87) International publication number: WO 2005/095986

(56) References cited:
- EP-A- 0 640 620
- BENGTSSON SARA H M ET AL: "Transcriptional regulation of the human carboxyl ester lipase gene in THP-1 monocytes: An E-box required for activation binds upstream stimulatory factors 1 and 2" BIOCHEMICAL JOURNAL, vol. 365, no. 2, 15 July 2002 (2002-07-15), pages 481-488, XP002342784 ISSN: 0264-6021
- HUI DAVID Y ET AL: "Carboxyl ester lipase: Structure-function relationship and physiological role in lipoprotein metabolism and atherosclerosis." JOURNAL OF LIPID RESEARCH, vol. 43, no. 12, December 2002 (2002-12), pages 2017-2030, XP002342785 ISSN: 0022-2275 cited in the application
- BRUNEAU NADINE ET AL: "Lectin-like Ox-LDL receptor is expressed in human INT-407 intestinal cells: Involvement in the transcytosis of pancreatic bile salt-dependent lipase." MOLECULAR BIOLOGY OF THE CELL, vol. 14, no. 7, July 2003 (2003-07), pages 2861-2875, XP002342786 ISSN: 1059-1524 cited in the application
- FALT HELEN ET AL: "Do human bile salt stimulated lipase and colipase-dependent pancreatic lipase share a common heparin-containing receptor?" ARCHIVES OF BIOCHEMISTRY AND BIOPHYSICS, vol. 386, no. 2, 15 February 2001 (2001-02-15), pages 188-194, XP002342787 ISSN: 0003-9861 cited in the application
- CAILLOL NATHALIE ET AL: "Pancreatic bile salt-dependent lipase activity in serum of normolipidemic patients" LIPIDS, vol. 32, no. 11, November 1997 (1997-11), pages 1147-1153, XP008052126 ISSN: 0024-4201 cited in the application
- SAXENA, U. ET AL.: 'Apoliprotein B en E basic amino acid clusters influence low-density lipoprotein association with lipoprotein lipase anchored to the subendothelial matrix' ARTERIOSCLER. THROMB. VASC. BIOL. vol. 15, 1995, pages 1240 - 1247, XP001013610
- SAXENA, U. ET AL.: THE JOURNAL OF BIOLOGICAL CHEMISTRY, vol. 268, no. 20, 1993, pages 14812-14819,
- HORIUCHI,S. ET AL.: AMINO ACIDS, vol. 25, 2003, pages 283-292,
- MORIWAKI, H. ET AL.: ARTERIOSCLEROSIS THROMBOSIS AND VASCULAR BIOLOGY, vol. 18, October 1998 (1998-10), pages 1541-1547,
- KODVAWALA, A. ET AL.: THE JOURNAL OF BIOLOGICAL CHEMISTRY, vol. 280, no. 46, 18 November 2005 (2005-11-18), pages 38592-38598,
- GUSTAFSSON, M. ET AL.: CMLS, vol. 61, January 2004 (2004-01), pages 4-9,
- PENTAKÄINEN, M.O. ET AL.: ARTERIOSCLEROSIS THROMBOSIS AND VASCULAR BIOLOGY, vol. 22, 2002, pages 211-217,
- REBAÏ, O. ET AL.: ARTERIOSCLEROSIS THROMBOSIS AND VASCULAR BIOLOGY, vol. 25, 2005, pages 359-364,
- CAMAROTA, L.M. ET AL.: THE JOURNAL OF BIOLOGICAL CHEMISTRY, vol. 279, no. 26, June 2004 (2004-06), pages 27599-27606,
- MCKILLOP, A.M. ET AL.: 'Characterization of the C-terminal region of molecular forms of human milk bile salt-stimulated lipase' ACTA PAEDIATR vol. 93, no. 1, 2004, pages 10 - 16, XP008061615
- STRÖMQVIST, M. ET AL.: ARCHIVES OF BIOCHEMISTRY AND BIOPHYSICS, vol. 347, no. 1, 1 November 1997 (1997-11-01), pages 30-36,

## Description

The invention relates to methods of screening test compounds for their ability to reduce retention of atherogenic lipoproteins in atherogenesis by measuring their activity as a modulator of the binding affinity of CEL (Carboxyl Ester Lipase) to various receptors. The disclosure also relates to the use of active modulators of the binding affinity of CEL to receptors for the prevention and treatment of atherosclerosis.

### BACKGROUND OF THE INVENTION

### Development of atherosclerotic lesions

The first visible signs of development of atherosclerotic lesions are the fatty streaks, which can occur already during foetal development (Napoli et al. 1997). Fatty streaks consist of lipid deposits underlying the endothelium of arteries and are formed when modified forms of LDL (Low density lipoprotein) accumulate inside cells. Uptake of unmodified LDL is mediated via the LDL receptor (LDLR), and the expression of the LDLR is regulated in a feedback regulatory pathway by the intracellular cholesterol levels to prevent the cell from becoming overloaded with cholesterol. Oxidative modification of the LDL particle changes its biochemistry and the specific properties of oxidised LDL (oxLDL) depend on the extent of modification. Minimally modified LDL can still be recognised by the LDLR through the normal pathway, but extensively modified LDL particles are instead taken up via scavenger receptors on macrophages and smooth muscle cells (Navab et al. 1996). Unrestricted uptake of modified forms of LDL via the scavenger receptor pathway along with phagocytosis leads to excessive lipid accumulation in macrophages and smooth muscle cells, causing them to take on the so called foam cell phenotype.

One of the key events in atherosclerosis is the infiltration of monocytes into the subendothelial space of the vessel wall, where they differentiate into macrophages. At the initial stages of plaque development, the recruitment of phagocytic cells to the arterial wall may have a protective effect by removing cytotoxic and proinflammatory oxLDL particles and apoptotic cells. However, as the pathogenic process progresses, more and more macrophages are recruited and start becoming transformed into lipid-laden foam cells. Migration of monocytes, which can transform into macrophages, into the arterial wall is likely to be stimulated in part by oxLDL, which can directly attract monocytes and also induce expression of chemotactic molecules (Navab et al. 1996).

### The response to retention hypothesis of atherosclerosis

Many of the processes implicated in the early stages of atherogenesis including endothelial damage, lipoprotein oxidation and macrophage and VSMC (vascular smooth muscle cells) proliferation are individually not sufficient to lead to lesion development. The response-to-retention hypothesis suggests that subendothelial retention of atherogenic lipoproteins is the trigger for all of these processes which are in fact normal physiological responses to the accumulation of lipids.
LDL retention in vascular sites prone to development of atheroma does not appear to be the result of increased influx but rather a result of reduced efflux of atherogenic lipoproteins (Schwenke et al. 1989). Normally, the efflux of lipoproteins from arterial tissue is in close equilibrium with delivery and it is only after persistent disequilibrium that arterial cholesterol accumulation occurs (Proctor et al. 2002). The retention involves specific changes in the composition of the structural elements of the extracellular matrix, mainly proteoglycans, and there are likely to be pre-existing local differences in the presence of retaining molecules in the vascular wall (Williams et al. 1995). Following binding of LDL to proteoglycans, the LDL particle undergoes several biologically important modifications including aggregation and oxidation. LDL-proteoglycan complexes show increased susceptibility to oxidation compared to unbound LDL under experimental conditions (Hurt-Camejo et al. 1992).

There is accumulating evidence that increased risk of atherosclerosis is not entirely explained by raised levels of plasma lipoprotein concentrations, but that the arterial wall has differential affinity for different lipoprotein subspecies. Characteristics such as lipoprotein size, density, lipid composition and apolipoprotein content all determine the extent of retention and associated inflammatory response (Proctor et al. 2002). There has recently been particular focus on the role of ApoB (Apolipoprotein B) as a mediating factor for retention of LDL and indeed, recent research show that both ApoB-48, which is associated with chylomicrons, and ApoB-100, which is associated with VLDL/LDL, contain proteoglycan-binding sites although some may be masked in vivo (Flood et al. 2002).

While the major determinant of initial retention of LDL is likely to be the proteoglycan composition within the subendothelial space, macrophage-derived lipoprotein lipase (LPL) may facilitate and enforce retention once the lesion has started to form (Pentikainen et al. 2002). In adipose and muscle tissue, the role of LPL is to hydrolyse the triglycerides of chylomicrons and VLDL (very low density lipoprotein) particles with release of free fatty acids. The vast majority of LPL is located in the capillary endothelium but a small fraction of LPL is also found in the arterial endothelium where it may create remnant particles that can be trapped within the intima (Pentikainen et al. 2002). The intimal LPL appears to be derived from macrophages and smooth muscle cells and is abundantly present throughout the extracellular matrix. The LPL that is bound to the components of the extracellular matrix can act as bridging molecules in the retention of LDL (Pentikainen et al. 2002).

### The lipolytic enzyme carboxyl ester lipase (CEL)

Carboxyl ester lipase (CEL), also referred to as bile salt stimulated lipase (BSSL) or bile salt stimulated cholesterol esterase, has a wide substrate specificity and can hydrolyse mono-, di-, and triacylglycerol, phospholipids and esters of fat soluble vitamins in addition to cholesteryl esters (Hui 1996). CEL is involved in the digestion and absorption of dietary lipids and seems to be primarily important for the uptake of cholesteryl esters (Howles et al. 1996). The enzyme is expressed at high levels in the exocrine pancreas and contributes up to as much as 5% of the total protein content of pancreatic juice (Wang and Hartsuck 1993; Hui and Howles 2002).

There are several potential functions for CEL in the intestinal tract suggested by in vitro studies, but only the cholesteryl ester hydrolytic activity is unique for CEL ( Hui and Howles 2002). The CEL knockout mouse (CELKO) displays normal growth and development and normal absorption of triglycerides, free cholesterol and retinyl esters (Weng et al. 1999). However, CELKO mice display a markedly reduced ability to absorb cholesteryl esters (Weng et al. 1999) indicating that CEL may be the primary enzyme involved in cholesteryl ester absorption. While there appears to be no quantitative differences in the total amount of free cholesterol absorbed by CELKO mice compared to control mice, a recent study suggests that there may be a qualitative difference. CELKO mice produce predominantly smaller lipoprotein particles compared to control mice, which primarily produce large chylomicrons and VLDL (Kirby et al. 2002). This suggests that CEL somehow influences the assembly and secretion of lipoproteins in the intestine. A wider role for CEL in lipid metabolism is implicated by the presence of CEL mRNA and activity in human plasma and aortic tissue (Shamir et al. 1996). A number of possible sources have been proposed including liver cells (Winkler et al. 1992), circulating monocytes (Li and Hui 1997), eosinophils (Holtsberg et al. 1995) and vascular endothelial cells (Li and Hui 1998). While the presence of CEL mRNA in vascular cells, such as monocytic cells and end othelial cells, suggests that CEL may be locally produced in the circulation or the vessel wall, the CEL detected in plasma may also originate from the pancreas after transcytosis through the intestines (Bruneau et al. 2003a). Like pancreatic and mammary gland-derived CEL, the macrophage-expressed CEL appears to be secretory as evidenced by bile salt dependent cholesteryl hydrolysis in conditioned medium from PMA stimulated cells of the human monocytic cell line THP-1 and monocyte derived macrophages (Li and Hui 1997).

### The CEL protein

The C-terminal part of CEL consists of a unique structure with proline-rich O-glycosylated repeats of 11 amino acid residues each (Nilsson et al. 1990; Reue et al. 1991) encoded in exon 11 of the CEL gene (Lidberg et al. 1992). The number of proline-rich repeats has been reported to vary extensively between species, ranging from three in mouse (Lidmer et al. 1995) to 16 in humans (Nilsson et al. 1990) and 39 in gorilla (Madeyski et al. 1999), while there are no repeats at all in salmon (Gjellesvik et al. 1994). This diversity in number of repeated units can explain the observed size differences between species; the mouse CEL is a 74kDa protein while the human CEL, which is extensively glycosylated across the repeated region, has an apparent molecular mass of 120-140 kDa (Blackberg and Hernell 1981; Wang and Johnson 1983). In addition to variations between species in the repeated region of the CEL gene, at least in the human gene there are also variations between individuals. This difference, which was first observed in CEL proteins secreted from the lactating mammary gland (Stromqvist et al. 1997), was subsequently found to be due to a variation in the number of repeated elements and in fact approximately 50% of the examined subjects were found to be carriers of alleles that deviate from the common 16 repeat-allele (Lindquist et al. 2002; Higushi et al. 2002).

While it is possible that the repeat polymorphism is merely a genetic marker for lipid profile, it is also possible that it has functional role in determining plasma lipid composition. It has been suggested that the repeats may have a functional role in protecting CEL from proteolytic degradation (Loomes et al. 1999) and that their O-glycosylation is important for secretion of the enzyme (Bruneau et al. 1997). On the other hand, the repeated region may not be important for catalytic activity, activation by bile salts and heparin binding (Hansson et al. 1993; Downs et al. 1994; Blackberg et al. 1995.

### The possible role of CEL in atherosclerosis

The finding that CEL is present in the circulation suggests that the enzyme may take part in processes outside the intestinal lumen. In vitro experiments have shown that CEL can modify the LDL and HDL lipid composition and reduce the atherogenicity of oxLDL by decreasing its lysophosphatidylcholine (lysoPC) content, which indicates that CEL may function as a protective factor in the development of atherosclerosis (Shamir et al. 1996). On the other hand CEL has been suggested to convert larger LDL particles to smaller more atherogenic subspecies (Brodt-Eppley et al 1995) which would instead render CEL pro-atherogenic in the vascular setting.

### CEL in atherosclerotic lesions

Research on the CEL knockout mice has focused mainly on the uptake of dietary lipids and serum levels of cholesterol and so far no studies have addressed the possibility of a vascular phenotype in these animals. The lack of attention to this interesting prospect may have arisen, in part, from the suggestion that CEL is not expressed in mouse macrophages and that instead hormone sensitive lipase may compensate for the lack of CEL in this species (Li and Hui 1997). However, this suggestion is based on the absence of detectable CEL mRNA transcripts in the mouse macrophage cell line J774 (Li and Hui 1997), and further studies on both mRNA as well as protein level in primary mouse macrophages are required to confirm this.

It has previously been shown that the serum levels of CEL correlate positively with serum levels of LDL (Brodt-Eppley et al. 1995; Caillol et al. 1997) and that circulating CEL may in fact be attached to LDL particles (Caillol et al. 1997). Furthermore, it has been suggested that, at least in rat, circulating CEL derives from the pancreas and is transported across the intestinal lumen and that in plasma it is associated with lipoproteins in the fraction containing chylomicrons, VLDL and LDL (Bruneau et al. 2003a).

In a recent study CEL was found to be associated with smooth muscle cells but not with macrophages in the aortic beds of cynomolgus monkeys (Auge et al. 2003). However, this study also show that CEL expression was not detected intracellularly in human smooth muscle cells, and the authors therefore draw the conclusion that the CEL appearing to be associated with smooth muscle cells in the aortic wall must be derived from an alternative source.

According to the response-to-retention hypothesis, the affinity by which LDL bind to vascular proteoglycans is a determinant of subendothelial retention. In light of the fact that CEL is present in LDL it is interesting to note that the N-terminal part of the CEL protein contains a region which binds avidly to heparin and several heparin variants (Falt et al. 2001). The binding of CEL to vascular proteoglycans remains to be thoroughly investigated.

In addition to this, it was recently suggested that the transport of CEL across intestinal enterocytes (Bruneau et al. 2001) in fact appears to be mediated by the lectin-like ox-LDL receptor (LOX-1) (Bruneau et al. 2003b). LOX-1 was originally identified as a receptor for oxLDL in endothelial cells (Sawamura et al. 1997) and macrophages (Moriwaki et al .1998) and it specifically recognises the protein moiety of oxLDL (Moriwaki et al. 1998). Because of its lectin-like structure in the extracellular domain, LOX-1 may interact with certain sugar chains on the protein portion of the modified LDL-particle (Moriwaki et al. 1998). LOX-1 is implicated as a scavenger receptor for oxLDL and has therefore been implicated in the lipid accumulation in atherosclerotic lesions. The role of retention of atherogenic lipoproteins in the development of atherogenesis has been reviewed (Gustafsson et al, 2004).

### SUMMARY OF THE INVENTION

The present invention is based on the discovery that CEL takes part in processes leading to the retention of atherogenic lipoproteins in atherogenesis.

The invention relates to the use of a variety of procedures for using CEL in the discovery of modulators of the retention of atherogenic lipoproteins in atherogenesis and the use of such modulators to prevent and treat atherosclerosis.

The disclosure also relates to pharmaceutical compositions containing such a modulator discovered by the methods described in this application and the use of the pharmaceutical composition comprising such modulator to reduce the retention of atherogenic lipoproteins in atherogenesis for the prevention and treatment of atherosclerosis.

### DETAILED DESCRIPTION OF THE INVENTION

The present inventors is based on the finding that modulators of the binding affinity of CEL to various receptors will reduce the retention of atherogenic lipoproteins in atherogenesis.

The present invention provides an in vitro method of identifying a compound useful for prevention and treatment of atherosclerosis which comprises assaying the compound for its ability to decrease the binding affinity of CEL to a receptor as defined in claim 1.

The present invention provides a method of identifying a compound useful for reducing the retention of atherogenic lipoproteins in atherogenesis which comprises assaying the compound for its ability to decrease the binding affinity of CEL to a receptor. The present disclosure further provides a method for reducing the retention of atherogenic lipoproteins in atherogenesis comprising the administration of an effective amount of a modulator of the binding affinity of CEL to a receptor.

In a further aspect of the present disclosure a method for the provision of an agent for the reduction of the retention of atherogenic lipoproteins in atherogenesis is provided, which method comprises using one or more putative modulator of the binding affinity of CEL to a receptor as test compounds in one or more procedures to measure the ability of the test compound to reduce the retention of atherogenic lipoproteins, and selecting an active compound for use as an agent able to reduce the retention of atherogenic lipoproteins in atherogenesis.

Convenient test procedures include the use of animal models to test the role of the test compound. These will typically involve the administration of compounds by intra peritoneal injection, subcutaneous injection, intravenous injection, oral gavage or direct injection via canullae into the blood stream of experimental animals.

Suitable putative modulators may be firstly identified by screening of test compounds using a method for the determining the ability of a test compound to modulate the binding affinity of CEL or fragment or chimeric form thereof to a suitable receptor.
Preferably the method for screening is selected from methods utilizing:
i) measurement of the affinity of CEL to a receptor using chromatographic methods with CEL or the receptor as the stationary phase
ii) measurement of binding of CEL to cells expressing the receptor on the cellular surface, preferably using labelled CEL
iii) measurement of binding of CEL to a receptor using scintillation proximity, ultracentrifugation, preferably using labelled CEL
iv) measurement of binding of CEL to vascular tissue, preferably using labelled CEL

Labelled CEL can be obtained using standard methods such as radiolabelling with ³H, ¹⁴C, ¹⁵N, ¹²⁵I, labelling with fluorophores such as FITC, rhodamine, or affinity labels such as avidin or biotin.

CEL can be detected by determining lipase activity, by CEL specific polyclonal or monoclonal antibodies.
Examples of suitable receptors are vascular proteoglycans such as glycosaminoglycans, heparin, heparan sulphate, chondroitin-6-sulphate, chondroitin-4-sulphate, dermatan sulphate; scavenger receptors such as SR-A types I, II and III, MARCO, SR-BI, CD36, SR-C1, SR-D, Macrosialin/CD86, SR-E, LOX-1 (lectin-like ox-LDL receptor), SR-F, SREC-1, SR-PSOX, FEEL-1, FEEL-2; AGE receptors such as RAGE, 80K-H, OST48, Galectin-3; (for a review of the different classes of scavenger and AGE receptors see Horiu chi et al. 2003); LPL (lipoprotein lipase); apolipoproteins such as apo A-I, apo A-II, ap o B-100, apo B-48. apo C-I, apo C-II, apo C-III, apo E; lipoproteins and lipoprotein particles such as the VLDLs (very low-density lipoproteins) VLDL1, VLDL2 and VLDL3, the IDLs (intermediate-density lipoproteins) IDL1, IDL2 and IDL3, LDLs (low density lipoproteins) LDL1, LDL2 and LDL3, the HDLs (high-density lipoproteins) preβ-HDL, α-HDL, HDL1, HDL2, and HDL3 (for a review of the different classes of lipoproteins see Tulenko and Sumner 2002).

CEL may be isolated from a suitable tissue such as pancreas or from milk. Alternatively recombinant CEL can be produced using standard methods through the isolation of DNA encoding CEL.

DNA encoding CEL may be conveniently isolated from commercia Ily available RNA, cDNA libraries, genomic DNA, or genomic DNA libraries using conventional molecular biology techniques such as library screening and/or Polymerase Chain Reaction (PCR). These techniques are extensively detailed in Molecular Cloning - A Laboratory Manual, 2nd edition, Sambrook, Fritsch & Maniatis, Cold Spring Harbor Press.

The resulting cDNAs encoding CEL are then cloned into commercially available mammalian expression vectors such as the pcDNA3 (Invitrogen), pMC1neo (Stratagene), pXT1 (Stratagene), pSG5 (Stratagene), EBO-pSV2-neo (ATCC 37593), pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), IZD35 (ATCC 37565), pLXIN, pSIR (CLONTECH), and pIRES-EGFP (CLONTECH). Standard transfection technologies are used to introduce the resulting expression vectors into commonly available cultured, mammalian cell lines such as L cells L-M(TK-) (ATCC CCL 1.3), L cells L-M (ATCC CCL 1.2), THP-1 (ATCC TIB 202), HEK 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26) and MRC-5 (ATCC CCL 171). CHO, HEK293, HeLa and clonal derivatives expressing the CEL are isolated. These transfected cell lines are used to produce recombinant CEL.

Alternatively the cDNAs encoding CEL are cloned into commonly available expression vectors suitable for expression in micro organisms, such as bacterial exp ression vectors such as the pET (Invitrogen), pDEST (Invitrogen), pLEX (Invitrogen), pCAL (Stratagene); and the yeast expression vectors pYES(Invitrogen), pESC (Stratagene) for expression in saccharomyces and pPICZ (Invitrogen) for expression in pichia. Standard transfection technologies are used to introduce the resulting expression vectors into commonly available strains of micro organisms, such as the E.coli strains JM101 (Stratagene) and JM110 (Stratagene).

Methods for purification of CEL from different tissues and transfected cell-lines are known in the art (Lombardo et al. 1978; Blackberg and Hernell 1981; Wang and Johnson 1983; Hansson et al. 1993)
The test compound may be a polypeptide of equal to or greater than, 2 amino acids such as up to 6 amino acids, up to 10 or 12 amino acids, up to 20 amino acids or greater than 20 amino acids such as up to 50 amino acids. Antibodies raised against or reacting with CEL may also be used as test compounds.

For drug screening purposes, preferred compounds are chemical compounds of low molecular weight and potential therapeutic agents. They are for example of less than about 1000 Daltons, such as less than 800, 600 or 400 Daltons in weight. If desired the test compound may be a member of a chemical library. This may comprise any convenient number of individual members, for example tens to hundreds to thousands to millions etc., of suitable compounds, for example peptides, peptoids and other oligomeric compounds (cyclic or linear), and template-based smaller molecules, for example benzodiazepines, hydantoins, biaryls, carbocyclic and polycyclic compounds (e.g. naphthalenes, phenothiazines, acridines, steroids etc.), carbohydrate and amino acids derivatives, dihydropyridines, benzhydryls and heterocycles (e.g. triazines, indoles, thiazolidines etc.). The numbers quoted and the types of compounds listed are illustrative, but not limiting. Preferred chemical libraries comprise chemical compounds of low molecular weight and potential therapeutic agents.

In a further aspect of the disclosure we provide the use of a modulator of the binding affinity of CEL to a receptor as an agent able to reduce the retention of atherogenic lipoproteins in atherogenesis and thereby preventing or reducing atherosclerosis.

In a further aspect of the present disclosure we provide a method of preventing or treating atherosclerosis which method comprises administering to a patient in need thereof a pharmaceutically effective amount of an agent, preferably identified using one or more of the methods of this invention, able to reduce the retention of atherogenic lipoproteins and thereby preventing or treating atherosclerosis.

This disclosure further provides use of an agent able to reduce the retention of atherogenic lipoproteins by modulating the binding affinity of CEL to a receptor in preparation of a medicament for the prevention or treatment of atherosclerosis.

According to another aspect of the present disclosure there is provided a method of preparing a pharmaceutical composition which comprises:
i) identifying an agent as useful for reducing the retention of atherogenic lipoproteins in atherogenesis according to a method as described herein; and
ii) mixing the agent or a pharmaceutically acceptable salt thereof with a pharmaceutically acceptable excipient or diluent.
It will be appreciated that the present invention includes the use of orthologues, homologues and genetic variants of the human CEL.

By the term "orthologue" we mean the functionally equivalent CEL in other species.

By the term "homologue" we mean a substantially similar and/or related CEL in the same or a different species.

By the term "genetic variant" we mean a variant of CEL resulting from a polymorphism in the CEL gene

The terms "prevention and treatment of atherosclerosis" is meant to include prevention and treatment of atherosclerosis related conditions and subsequent complications, exemplified by but not limited to,
i) coronary artery disease, including related complications such as angina pectoris, myocardial infarction, and congestive heart failure,
ii) cerebrovascular disease, including extracranial carotid arterial disease, including related complications such as transient ischemic attack (TIA), and stroke,
iii) peripheral vascular diseases, including related complications such as, arterial rupture, organ damage such as kidney damage, intestinal da mage, claudication, including intermittent claudication, gangrene, and erectile dysfunction,
iv) complications after invasive procedures such as restenosis after percutaneous transluminal coronary angioplasty (PTCA) or cutting balloon angioplasty (CBA), and occlusion of bypass grafts/stent thrombosis;
as well as preventive treatment of patients in risk groups, exemplified but not limited to, patients with type II diabetes, obesity, hypertension, dyslipidemias, risk associated genotypes such as certain LPL (lipoprotein lipase) genotypes, LDL-receptor genotypes, and apo-B genotypes, and smokers.

For either of the above definitions the CEL may have for example at least 30%, such as at least 40%, at least 50%, at least 60%, and in particular at least 70%, such as at least 80%, for example 85%, or 90% or 95% peptide sequence identity with the human CEL having the amino acid sequence which can be obtained from the SwissProt database, accession no P19835 (CEL_HUMAN).

To determine the percent identity of two amino acid sequences or of two nucleic acids, the sequences are aligned for optimal comparison purposes (e.g., gaps can be introduced i n the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., percent identity = number of identical positions/total number of positions (e.g., overlapping positions) x 100). In one embodiment, the two sequences are the same length.

To determine percent sequence identity, a target nucleic acid or amino acid sequence is compared to the identified nucleic acid or amino acid sequence using the BLAST 2 Sequences (Bl2seq) program from the stand-alone version of BLASTZ containing BLASTN version 2.0.14 and BLASTP version 2.0.14. This stand-alone version of BLASTZ can be obtained from the U.S. government's National Center for Biotechnology Information web site (world wide web at ncbi.nlm.nih.gov). Instructions explaining how to use the Bl2seq program can be found in the readme file accompanying BLASTZ.

Bl2seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); - q is set to -1; -r is set to 2; and all other options are left at their default setting. The following command will generate an output file containing a comparison between two sequences: C:\Bl2seq -i c:\seq1.txt -j c:\seq2.txt -p blastn -o c:\output.txt -q -1 -r 2. If the target sequence shares homology with any portion of the identified sequence, then the designated output file will present those regions of homology as aligned sequences. If the target sequence does not share homology with any portion of the identified sequence, then the designated output file will not present aligned sequences.

Once aligned, a length is determined by counting the number of consecutive nucleotides from the target sequence presented in alignment with sequence from the identified sequence starting with any matched position and ending with any other matched position. A matched position is any position where an identical nucleotide is presented in both the target and identified sequence. Gaps presented in the target sequence are not counted since gaps are not nucleotides. Likewise, gaps presented in the identified sequence are not counted since target sequence nucleotides are counted, not nucleotides from the identified sequence.

The percent identity over a particular length is determined by counting the number of matched positions over that length and dividing that number by the length followed by multiplying the resulting value by 100. For example, if (1) a 50 nucleotide target sequence is compared to the sequence encoding human CEL (2) the Bl2seq program presents 45 nucleotides from the target sequence aligned with a region of the sequence encoding human CEL where the first and last nucleotides of that 45 nucleotide region are matches, and (3) the number of matches over those 45 aligned nucleotides is 40, then the 50 nucleotide target sequence contains a length of 45 and a percent identity over that length of 89 (i.e., 40 / 45 x 100 = 89).

It is appreciated that homologous CEL may have substantially higher peptide sequence identity over small regions representing functional domains. We include CEL having greater diversity in their DNA coding sequences than outlined for the above amino acid sequences but which give rise to CEL having peptide sequence identity falling within the above sequence ranges. Convenient versions of the CEL include the published sequence. The amino acid sequence of human CEL can be obtained from the SwissProt database, accession no P19835 (CEL_HUMAN) and the cDNA sequence e.g. from the EMBL database accession no. X54457. The CEL is from any mammalian species, including human, monkey, rat, mouse and dog. Preferably the human CEL is used.

Fragments and partial sequences of CEL may be useful in the assays and analytical methods of the invention. It will be appreciated that the only limitation on these is practical, they must comprise the necessary functional elements for use in the relevant assay and/or analytical procedures. The agent of this disclosure may be administered in standard manner for the condition that it is desired to treat, for example by oral, topical, parenteral, buccal, nasal, or rectal administration or by inhalation. For these purposes the compounds may be formulated by means known in the art into the form of, for example, tablets, capsules, aq ueous or oily solutions, suspensions, emulsions, creams, ointments, gels, nasal sprays, suppositories, finely divided powders or aerosols for inhalation, and for parenteral use (including intravenous, intramuscular or infusion) sterile aqueous or oily solutions or suspensions or sterile emulsions.

### LEGENDS TO FIGURES

*Figure 1*
   Paraffin embedded sections of atherosclerotic human carotid artery were used for immunohistochemistry. Positive staining for CEL (top left) overlaps with CD68 staining for macrophages (top right). Positive staining for smooth muscle marker α-actin, not coinciding with CEL staining, is shown in the bottom left panel and a negative control created by omission of the primary antibody is shown bottom right.
*Figure 2*
   CEL co-localizes with macrophages by double immunofluorescence staining. Red fluorescence shows CEL and green fluorescence show macrophages (CD68) in top panel and smooth muscle cells (α-actin) in bottom panel.
*Figure 3*
   CEL protein is present in cell lysate from primary monocytes (lane 5) and monocyte-derived macrophages (lanes 1 and 2) as well as in cell culture media collected from the macrophages (lanes 3 and 4). Purified CEL protein (1ng) is used as a positive control (lane 6).
*Figure 4*
   Western blot and RT-PCR analysis of CEL in macrophages. Panel A shows a Western blot analysis of cell culture media collected from primary monocytes, differentiated into macrophages for up to nine days, at the indicated time points. The "medium" lane represents a control sample of cell culture medium that had not been in contact with the cells. Panel B shows cell lysate samples from fully differentiated monocyte-derived macrophages treated with LPS (1µg/ml), IFN-γ(200U/ml) or oxLDL (50µg protein/ml) for 24 hours. The corresponding analysis of CEL mRNA by RT-PCR is shown in panel C. Amplification of GAPDH ensured equal amounts of RNA in the samples. The CEL primers were designed to yield a 579 bp product with amplification of cDNA and a 2443 bp product in the case of amplification of contaminating genomic cDNA. The GAPDH primers yield a 597 bp product when amplifying cDNA and 1090 bp when amplifying genomic DNA. No amplification of genomic DNA was observed.
*Figure 5*
   CEL is abundant in LDL fractions of human serum. Panel A shows intense staining for CEL and apoB in the core region of a plaque in an atherosclerotic carotid artery. Panel B shows a Western blot with chylomicron-rich serum from two donors (2µg protein/lane). While CEL is absent in the chylomicron/VLDL fraction it is abundant in the HDL/LDL fraction. Panel C shows HDL/LDL samples corresponding to 2µg protein/lane (CEL top panel) or 20µg/lane (apoB bottom panel). CEL is abundantly present in the HDL/LDL fraction of human serum in a pattern that follows that of apoB (bottom panel). The positive control lane was loaded with 1ng purified CEL protein.

### EXAMPLES

### Example 1. CEL co-localizes with macrophages in human atherosclerotic lesion

### Methods

Immunohistochemical analysis. Paraffin embedded 6 µm sections of human atherosclerotic carotid artery (obtained according to protocols approved by the Ethics Research Committee at the Sahlgrenska University Hospital, Göteborg, Sweden) were used in the immunohistochemistry analysis. Paraffin embedded sections were cleared in xylene and rehydrated through an alcohol series and unmasking of epitopes was performed by 10 min pressure-boiling of the sections in Tris-EDTA buffer. Prior to addition of primary antibodies, the sections were treated with normal goat-serum and Avidin/Biotin Blocking kit (Vector Laboratories Inc.). Endogenous peroxidase activity was blocked by incubation with 0.3% hydrogen peroxide before the sections were incubated with either anti-CEL (polyclonal, specific for the C-terminal part of CEL, Stromqvist et al. 1997), anti-CD68 (KP1, DakoCytomatation), anti-alpha-actin (mouse monoclonal clone 1A4, Abcam) or anti-apoB (s-a-LDL, sheep antiserum against human apolipoprotein B). Species-appropriate biotinylated secondary antibodies were applied and the sections were incubated with avidin-biotin-peroxidase complex (Vectastain ABC kit, Vector Laboratories Inc.). Co-localization was verified by double immunostaining. Anti-CEL was visualized with biotinylated goat-anti-rabbit secondary antibodies followed by Texas-red conjugated streptavidin. After application of Avidin/Biotin Blocking kit (Vector Laboratories Inc.) anti-CD68 and anti-alpha actin were visualized with species-appropriate biotinylated secondary antibodies followed by streptavidin-FITC. VectaShield (Vector Laboratories Inc.) was used for mounting and sections were viewed under a fluorescence equipped Zeiss Axioplan2 Imaging.

Macrophage cell culture. Mononuclear cells were isolated through a Ficoll-Hypaque discontinuous gradient (Boyum et al. 1976) from buffy coats obtained from the local blood bank (Sahlgrenska University Hospital, Göteborg, Sweden). The cells were resuspended in RPMI 1640 medium (PAA Laboratories GmbH, Pasching, Austria) supplemented with 2 mmol/l glutamine, 2 mmol/l nonessential amino acids, 100 U/ml penicillin, 100 µg/ml streptomycin, and 20 mmol/l sodium pyruvate. Monocytes were obtained by allowing cells to adhere to plastic for 60 min at 37°C. Non-adhered cells were removed by washing with PBS and the adhered monocytes were used immediately or after 4 hours for experiments or left to differentiate into macrophages in supplemented RPMI1640 containing 20% heat-inactivated human serum for up to 9 days. Macrophages were then approximately 95% pure, as judged by flow cytometry. For experiments the cells were incubated for 24 h with supplemented RPMI1640 (pH 7.4) with 1 µg/mL lipopolysaccaride (LPS), interferon-gamma (γ-IFN) 200 U/mL (Sigma Aldrich, Stockholm, Sweden). The viability of cells was >95% in all incubations, as determined by Trypan blue exclusion.

Exposure of macrophages to oxidatively modified LDL. Fresh human EDTA-plasma was obtained from healthy male volunteers after overnight fasting. LDL (density 1.019-1.063g/L) was isolated by sequential ultracentrifugation (Olofsson et al. 1980). Before oxidation, native LDL was desalted on a PD-10 column (Amersham Biosciences), equilibrated with PBS containing penicillin 100 µg/mL and streptomycin 100 µg/ml (PEST) and using PBS-PEST as elution buffer. Oxidized LDL was generated by oxidation of LDL in PBS containing 5 µM CuSO₄ at 37°C. Oxidation was interrupted by adding 0.5 mmol/L EDTA. The oxLDL was purified on a PD-10 column, with PBS as elution buffer and sterilized by filtration through a 0.22 µm filter. Macrophages were incubated with oxLDL (50µg protein/ml) for 24 h. Protein concentration of lipoproteins was determined with the BioRad protein assay using γ-globulin as standard.

Isolation and analysis of RNA. Total RNA was isolated from monocytes and macrophages using the RNeasy-kit (Qiagen). 0.5 µg of each RNA sample was reverse transcribed in a total volume of 20 µl, using the SuperScript™ First-strand synthesis for RT-PCR (Invitrogen Life Technologies) and used in subsequent PCR-reactions. The PCR reactions were performed using the HotMaster™Taq polymerase (Eppendorf) with the following primers used for amplification of CEL 5'- agcacctacggggatgaaga-3' and 5'- gggctcggggatcagtaacct-3' and for house-keeping gene GAPDH 5'- ccacccatggcaaattccatggca -3' and 5'- tctagacggcaggtcaggtccacc -3'. Amplification of cDNA with the CEL-primers will yield a fragment of 579 bp while amplification of genomic DNA will yield a fragment of 2443 bp. Amplification of cDNA with GAPDH-primers will yield a fragment of 597 bp and while amplification of genomic DNA will yield a fragment of 1090 bp. For each PCR reaction 6 µl of the reverse-transcribed RNA was added and the following cycling parameters were used; initial denaturation at 96°C for 2 min followed by 30 cycles of 94°C for 30s, 59°C for 45s and 65°C for 2 min.

Fractionation of lipoproteins. For detection of CEL with Western blot, isolation of lipoprotein fractions was performed with two different methods. Isolation of very low density lipoprotein (VLDL) and low density lipoprotein (LDL) from serum was performed with preparative ultracentrifugation (Carlson 1973). VLDL was collected from the supernatant (density of 1.006 g/L). The infranatant contain LDL and high density lipoprotein (HDL).

Western blot analysis. Protein extracts from cultured cells, conditioned media from monocyte/macrophage cultures or serum lipoprotein fractions were electrophoresed through a pre-cast NuPage™ 4-12% Bis-Tris Gel (Invitrogen Life Technologies) and electro blotted onto Hybond-P filters (Amersham Biosciences). The filters were incubated with primary antibodies against CEL and apoB diluted in 5% milk TBS-Tween blocking solution. The primary antibodies were detected with POD-conjugated secondary IgG and ECL™ Western Blotting Detection Reagents (Amersham Biosciences), visualized on ECL™ films (Amersham Biosciences).

### Results

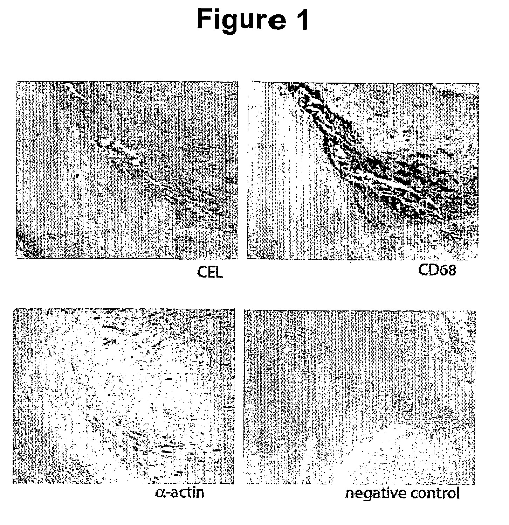

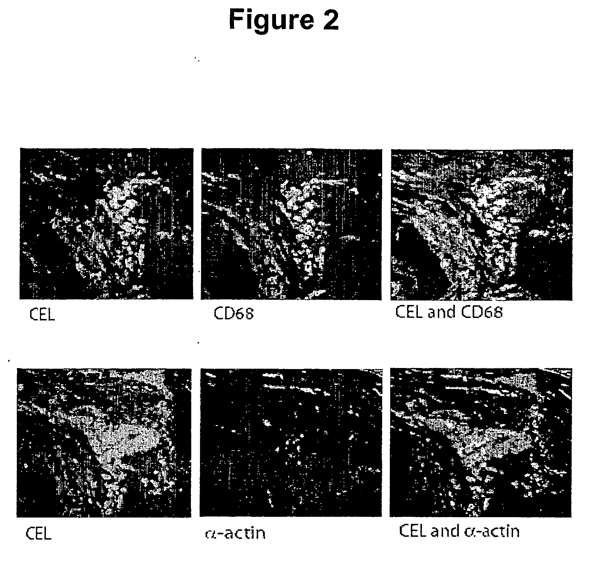

CEL is present in atherosclerotic carotid arteries. In sections of human atherosclerotic carotid arteries CEL was detected by labeling with CEL antibodies. Sections from carotid arteries of three individuals were stained for presence of CEL, CD68, and alpha-actin and representative pictures are shown in figure 1. The CEL-staining was mainly located to the lesion regions where it was present both inside cells as well as in the extra cellular regions, which is consistent with CEL being a secreted enzyme (figure 1 top left). Antibodies directed against the macrophage marker CD68 showed staining in the same region as CEL with intense staining in the core of the lesion, while the smooth muscle cell marker alpha-actin stained muscle cells around the lesion region (figure 1).
The DAB-staining of consecutive sections suggested that there is an overlap between the regions of staining for macrophages and CEL so to verify this overlap, double staining of atherosclerotic carotid artery sections with a combination of either CEL and CD68 or CEL and alpha-actin was performed. Representative pictures are shown in figure 2, revealing co-staining between CEL and CD68 staining but not between CEL and alpha-actin staining.

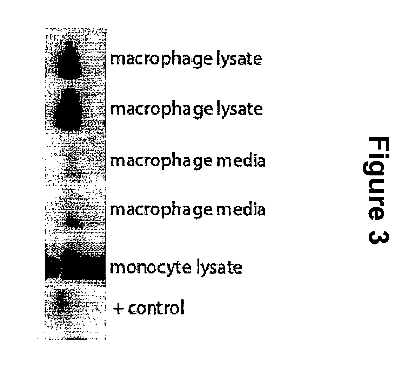

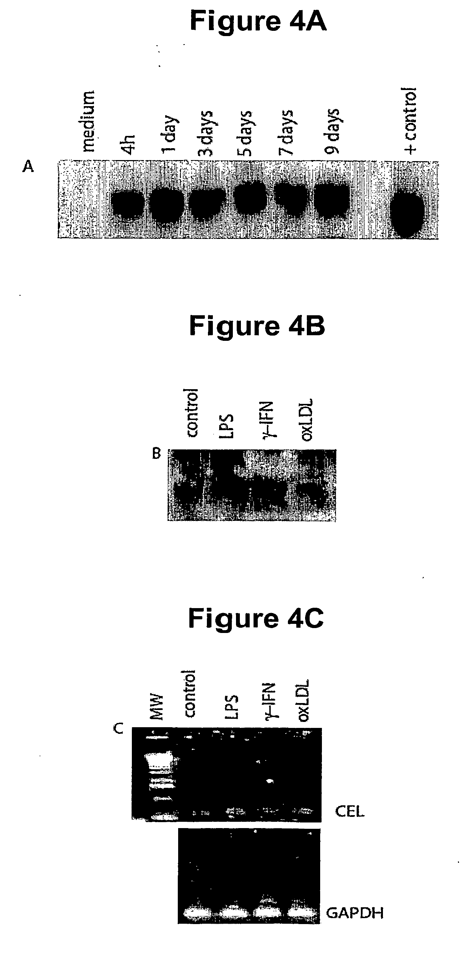

Human primary monocytes and macrophages express and secrete CEL. The immunohistochemical analysis indicated that macrophages may be the source of vascular CEL, at least in atherosclerotic tissue. The previous suggestions that human macrophages express CEL was based on the presence of CEL mRNA in these cells and CEL activity in conditioned media of cultured cells (Li and Hui 1997). This is supported by our finding that CEL is expressed in and secreted from human primary monocytes and macrophages. We used RT-PCR as well as western blot analysis to verify the expression and secretion. CEL mRNA transcripts were identified in both primary monocytes and in monocytes that had been treated with PMA to induce a macrophage-like phenotype (data not shown). Western blot analysis of proteins obtained by lysis of monocytes and monocyte-derived macrophages revealed detectable amounts of CEL protein (figure 3). To further characterize the expression of CEL by monocytes and macrophages we performed a time-course experiment to investigate if there is an up-regulation of CEL over time when primary monocytes assume a macrophage phenotype in culture. A Western blot experiment with protein collected from the culture media after 4 hours, and 1, 3, 5, 7 and 9 days in culture was performed. As can be seen in figure 4A there is no apparent up-regulation of CEL secretion over this time when the monocytes are differentiated into macrophages. Furthermore, to assess the possibility that CEL is up-regulated in response to atherosclerotic stimuli, we treated macrophages cultured for 7 days with lipopolysaccharide (LPS), interferon-gamma (□-IFN) or ox-LDL for 24 hours. The experiment was repeated with cells from four different donors but we could not detect any consistent increase or decrease in CEL expression at either the protein level (figure 4B) or at the mRNA level (figure 4C).

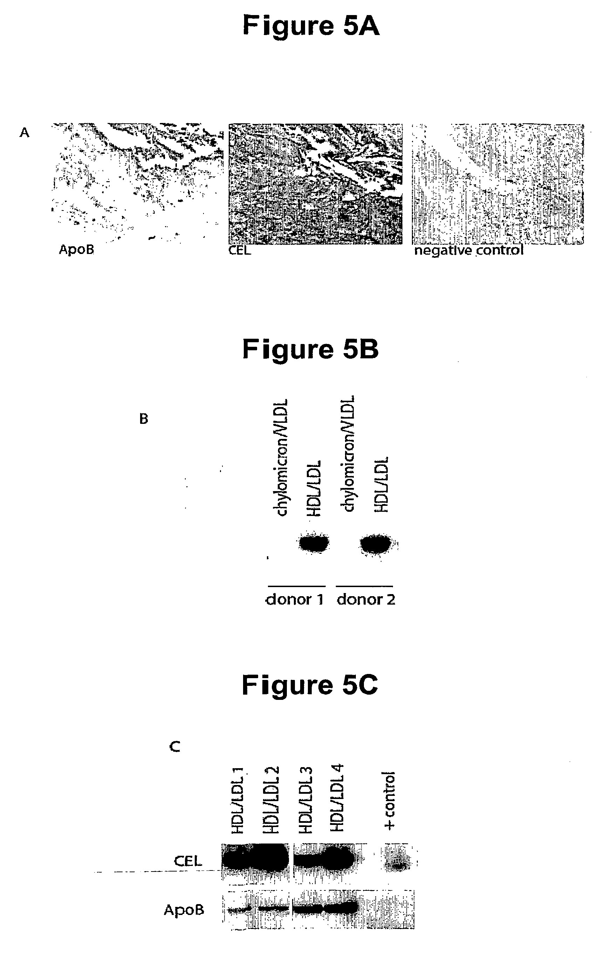

CEL is present in LDL-fractions from donor serum. The apparent accumulation of CEL protein in atherosclerotic lesion regions can be a consequence of the macrophage secretion of CEL within the lesion, but an alternative plausible explanation could be that the CEL is transported to the lesion site via the circulation. There have been suggestions in the literature that CEL may in fact be associated with apoB in the circulation (Bruneau et al. 2003a; Caillol et al. 1997). Immunohistochemical analysis revealed that CEL and apoB staining appear in the same regions of the atherosclerotic carotid artery (figure 5A), giving support to this possible alternative route of CEL accumulation. To further assess this, we obtained lipid fractions from serum donors and used Western blot to investigate the presence of CEL in chylomicron/VLDL, and HDL/LDL lipid fractions. Serum from two donors with high chylomicron content revealed that while CEL is present in the HDL/LDL-fractions it is seemingly absent in the chylomicron/VLDL-fractions (figure 5B). CEL was abundant in the HDL/LDL fractions, and the amount of CEL appeared to be to a certain extent correlated to the amount of apoB detected in the fraction (figure 5C).

### Discussion

The origin of circulating CEL is not well defined and there are a variety of sources from which it may be derived, including the pancreas after transcytosis through the intestines (Bruneau et al. 2003a). In this study we show that human primary monocytes and macrophages express and secrete CEL, and furthermore that immunostaining for CEL proteins in atherosclerotic regions co-localizes with immunostaining for the macrophage marker CD68. While our results suggest that monocytes/macrophages are possible sources of vascular CEL, we also took into account the notion that CEL can be transported in association with blood LDL (Calliol et al. 1997) to the vessel wall. We show using Western blot analysis of lipid fractions from serum donors that CEL is present in the apoB-containing LDL fractions, which is consistent with the immunostaining of atherosclerotic lesions showing presence of apoB in the same regions as CEL in the atherosclerotic lesions. Based on this, it is therefore reasonable to assume that there are at least two likely sources of the CEL that appears to accumulate in the atherosclerotic lesions. Interestingly, we could not detect any presence of CEL in the lipid fractions containing VLDL and chylomicrons. While the implications of this finding remain to be further explored it suggests that the CEL associated with serum lipoproteins is not simply transported along with chylomicrons from the intestinal tract, but becomes associated with lipoprotein particles at a later stage in the circulation.

Previous findings that CEL is present in the human aorta and that it has the ability to modify the LDL and HDL composition and reduce the atherogenicity of oxLDL by decreasing its lysophosphatidylcholine (lysoPC) content (Shamir et al. 1996), invoked a potential new role for CEL as a protective factor in the development of atherosclerosis. LysoPC acts as a chemoattractant for monocytes (Quinn et al. 1988), induces monocyte adhesion to the vascular endothelium and promotes macrophage proliferation (Sakai et al. 2000), which eventually leads to foam cell formation (Ross 1993). Due to its effects on lysoPC, it has been suggested that CEL may interact with cholesterol and oxidized lipoproteins to modulate the progression of atherosclerosis (Li and Hui 1997; Li and Hui 1998; Shamir et al. 1996).

One important process that has been implicated in the early stages of atherosclerosis is subendothelial retention of atherogenic lipoproteins (reviewed In William and Tabas 1995).

ApoB is the apolipoprotein associated with the atherogenic lipoprotein LDL, and it has been suggested that the atherogenicity of apoB-containing LDL may be linked to the affinity of apoB for artery wall proteoglycans, leading to retention of the LDL particle in the subendothelial space (Skalen et al. 2002). The binding of apoB-containing lipoproteins to extracellular proteoglycans is facilitated by lipoprotein lipase (LPL) which acts as a molecular bridge between the subendothelial proteoglycans and lipoproteins (Pentikainen et al. 2002).

The notion that affinity to vascular proteoglycans is a determinant of subendothelial retention invokes another potential role for the CEL associated with LDL. The N-terminal part of the CEL protein contains a region which binds avidly to heparin and several heparin variants (Fait et al. 2001), and while the binding of CEL to vascular proteoglycans remains to be investigated, CEL emerges a possible candidate for another bridging molecule. Furthermore, it was recently discovered that CEL can be transported across intestinal enterocytes in a transcytotic fashion via clathrin-coated pits (Bruneau et al. 2001), and that this transport appears to be mediated by the lectin-like ox-LDL receptor (LOX-1) (Bruneau et al. 2003b). LOX-1 was originally identified as a receptor for ox-LDL in endothelial cells (Sawamura et al. 1997) and macrophages (Moriwaki et al. 1998) and it specifically recognizes the protein moiety of ox-LDL (Moriwaki et al. 1998). Because of its lectin-like structure In the extracellular domain, LOX-1 may interact with certain sugar chains on the protein portion of the modified LDL-particle (Moriwaki et al. 1998). The fact that LOX-1 is implicated as a scavenger receptor for oxLDL as well as a putative receptor for CEL in intestinal cells provides another possible clue to why and how CEL accumulates in the vascular wall.

### Example 2. Affinity chromatography of CEL on proteoglycan columns.

Preparation and Characterization of Aortic Proteoglycans. Proteoglycans from intima-media of human aortas will be prepared by extraction from the intima-media at 4°C for 24h with 15 volumes of 6 M urea, 1 M NaCl in the presence of 10 mM EDTA, 10 mM ε-aminocaproic acid, 0.2 mM phenylmethyl sulfonyl fluoride, and 0.02% (w/v) NaN₃. After extraction, the mixture is centrifuged at 100,000 × *g* for 60 min. The supernatant is diluted with 6 M urea to give a final concentration of 0.25 M NaCl and loaded on a HiTrap Q column (Amersham Biosciences) (5 ml) equilibrated with 6 M urea, 0.25 M NaCl, 10 mM CaCl₂, and 50 mM acetate, pH 6.2, and the protease inhibitors. The column is washed with the above buffer, and the proteoglycans are eluted with a linear gradient of 0.25 to 1.0 M NaCl in the buffer (120 ml) at a flow rate of 2 ml/min. The eluted human arterial proteoglycans are collected, dialyzed against water, and lyophilized.

### Affinity Chromatography of CEL on a Proteoglycan, Chondroitin-6-Sulfate, or Heparin Affinity

Column. Human arterial proteoglycans or chondroitin-6-sulfate (Seikagaku Kogyo, Tokyo, Japan) are coupled to an N-hydroxysuccinimide-activated HiTrap column (Amersham Biosciences) according to the manufacturer's instructions. For this purpose, 1.0 mg of proteoglycans or 10 mg of chondroitin-6-sulphate in 0.2 M NaHCO₃ and 0.5 M NaCl, pH 8.3, are coupled to the column at 25°C for 2 h. The column is blocked with 0.5 M ethanolamine, pH 8.3, containing 0.5 M NaCl. The columns are equilibrated with buffer B (10 mM HEPES, 2 mM CaCl₂, 2 mM MgCl₂, pH 7.4) before use.

CEL is analyzed on the proteoglycan, chondroitin-6-sulphate or HiTrap heparin (Amersham Biosciences) affinity columns by elution with a linear gradient of NaCl (0 to 250 mM or 0 to 500 mM, in 10 min) in buffer B. Chromatography is performed at a flow rate of 1-2 ml/min. CEL is detected by UV absorbance at 280 nm. Retention of CEL on the affinity column is used as a measure of the affinity of CEL to the proteoglycan.

Test compounds are added together with CEL and evaluated for the ability to modulate the affinity of CEL to the proteoglycans.

### Example 3. Affinity of CEL to scavenger receptors

Expression of scavenger receptors in transfected cell-lines. DNA encoding the selected scavenger receptor, e.g. LOX-1, SR-A type I or II, SR-BI, is inserted into a suitable expression vector: The expression vector is used to transfect a suitable cell-line, e.g. CHO-K1, C-127, 3T3, HEK 293, THP-1. Cells are screened for intracellular uptake of labelled ox-LDL and positive cells are selected and expanded. Cell-lines stably expression the scavenger receptor are used for further experiments.
Measurement of the affinity of CEL to the scavenger receptor. Binding of CEL to transfected cells is measured after incubation with CEL and subsequent washings. The amount of bound CEL is determined using a CEL-antibody. Alternatively, labelled CEL, ¹²⁵I or FITC labelled, is used in the incubation and bound CEL is subsequently determined by measurement of bound radioactivity or fluorescence. The amount bound CEL is used as a measure of the affinity of CEL to the scavenger receptor. Un-transfected cells are used as control.

Test compounds are added together with CEL and evaluated for their ability to modulate the affinity of CEL to the scavenger receptors.

### Example 4. Affinity of CEL to lipoprotein particles

Isolation of lipoprotein fractions. Lipoprotein fractions are isolated from plasma of normal human volunteers using density gradient centrifugation. Whole plasma, with or without the addition of CEL, is loaded into a discontinuous NaCl/KBr gradient (1.3 and 1.006 g/ml), which forms into a linear gradient between upper and lower density limits during centrifugation for 150 min at 160,000 × *g*. The density ranges of the low and high density lipoprotein classes are 1.019-1.063 and 1.063-1.21 g/ml, respectively. The lipoproteins are de-salted on a G25 column (Amersham Biosciences).

Analysis of CEL in lipoprotein fractions. Each lipoprotein fraction is analyzed for the presence of co-migrating CEL. The analysis is performed e.g. by dot blotting or Western blotting using CEL specific antibodies. The amount of CEL in the lipoprotein fractions is used as a measure of the affinity of CEL to the specific lipoprotein present in the fraction.

Test compounds are added to the plasma samples before isolation of the lipoprotein fractions and evaluated for their ability to modulate the affinity of CEL to the different lipoproteins.

### Example 5. Affinity of CEL to lipoproteins

Immobilization of CEL. Purified CEL is immobilized on CnBr-activated Sepharose (Amersham Biosciences) (1 mg of protein/0.5 g of wet-gel) in 0.1 M sodium borate buffer (0.25 M NaCl, 5 mM CaCl₂ pH 8.0). After an overnight incubation at 4°C under agitation, the gel was allowed to settle and quenched with 10 gel volumes of 0.1 M ethanolamine (4 h, 4 C). Before use, the gel with immobilized CEL is washed alternatively with basic (0.1 M sodium phosphate, pH 8.0) and acidic (0.1 M MES, pH 5.5) buffers.

Affinity chromatography of lipoproteins. Plasma samples or isolated lipoproteins are chromatographed on an affinity column made of CEL immobilized on Sepharose gel to isolate lipoproteins that bind to CEL. After elution of unbound fraction with 5 mM MES, pH 6.0, 0.1 M NaCl, and 1 mM EDTA and washing of the column with the same buffer at pH 5.0, bound proteins were eluted at pH 8.0 (0.1 M sodium phosphate, 0.15 M NaCl). The eluted proteins are electrophoresed on SDS-PAGE and the isolated lipoproteins identified and quantified by Western blotting. The amount of the different lipoprotein isolated is used as a measure of the affinity of CEL to the specific lipoprotein.
Test compounds are added to the samples before chromatography on the affinity column made of immobilized CEL and evaluated for their ability to modulate the affinity of CEL to the different lipoproteins.

### REFERENCES

Auge, N., Rebai, O., Lepetit-Thevenin, J., Bruneau, N., Thiers, J.C., Mas, E., Lombardo, D., Negre-Salvayre, A., and Verine, A. 2003. Pancreatic bile salt-dependent lipase induces smooth muscle cells proliferation. Circulation 108:86-91.
Blackberg, L., and Hernell, O. 1981. The bile-salt-stimulated lipase in human milk. Purification and characterization. Eur J Biochem 116:221-225.
Blackberg, L., Stromqvist, M., Edlund, M., Juneblad, K., Lundberg, L., Hansson, L., and Hernell, O. 1995. Recombinant human-milk bile-salt-stimulated lipase. Functional properties are retained in the absence of glycosylation and the unique proline-rich repeats. Eur J Biochem 228:817-821.
Boyum A, Lovhaug D, Boecker WR. 1976. Regulation of bone marrow cell growth in diffusion chambers: the effect of adding normal and leukemic (CML) polymorphonuclear granulocytes. Blood 48:373-83.
Brodt-Eppley, J., White, P., Jenkins, S., and Hui, D.Y. 1995. Plasma cholesterol esterase level is a determinant for an atherogenic lipoprotein profile in normolipidemic human subjects. Biochim Biophys Acta 1272:69-72.
Bruneau, N., Nganga, A., Fisher, E.A., and Lombardo, D. 1997. O-Glycosylation of C-terminal tandem-repeated sequences regulates the secretion of rat pancreatic bile salt-dependent lipase. J Biol Chem 272:27353-27361
Bruneau, N., Nganga, A., Bendayan, M., and Lombardo, D. 2001. Transcytosis of Pancreatic Bile Salt-Dependent Lipase through Human Int407 Intestinal Cells. Exp Cell Res 271:94-108.
Bruneau, N., Bendayan, M., Gingras, D., Ghitescu, L., Levy, E., and Lombardo, D. 2003a. Circulating bile salt-dependent lipase originates from the pancreas via intestinal transcytosis. Gastroenterology 124:470-480.
Bruneau, N., Richard, S., Silvy, F., Verine, A., and Lombardo, D. 2003b. Lectin-like Ox-LDL receptor is expressed in human INT-407 intestinal cells: involvement in the transcytosis of pancreatic bile salt-dependent lipase. Mol Biol Cell 14:2861-2875.
Caillol, N., Pasqualini, E., Mas, E., Valette, A., Verine, A., and Lombardo, D. 1997. Pancreatic bile salt-dependent lipase activity in serum of normolipidemic patients. Lipids 32:1147-1153.
Carlson K. 1973. Lipoprotein fractionation. J Clin Pathol Suppl (Assoc Clin Pathol). 5:32-7.
Downs, D., Xu, Y.Y., Tang, J., and Wang, C.S. 1994. Proline-rich domain and glycosylation are not essential for the enzymic activity of bile salt-activated lipase. Kinetic studies of T-BAL, a truncated form of the enzyme, expressed in Escherichia coli. Biochemistry 33:7979-7985.
Falt, H., Hernell, O., and Blackberg, L. 2001. Do human bile salt stimulated lipase and colipase-dependent pancreatic lipase share a common heparin-containing receptor? Arch Biochem Biophys 386:188-194
Flood, C., Gustafsson, M., Richardson, P.E., Harvey, S.C., Segrest, J.P., and Boren, J. 2002. Identification of the proteoglycan binding site in apolipoprotein B48. J Biol Chem 277:32228-32233.
Gjellesvik, D.R., Lorens, J.B., and Male, R. 1994. Pancreatic carboxylester lipase from Atlantic salmon (Salmo salar). cDNA sequence and computer-assisted modelling of tertiary structure. Eur J Biochem 226:603-612. Gustafsson, M. et al. 2004. CMLS 61:4-9.
Hansson, L., Blackberg, L., Edlund, M., Lundberg, L., Stromqvist, M., and Hernell, O. 1993. Recombinant human milk bile salt-stimulated lipase. Catalytic activity is retained in the absence of glycosylation and the unique proline-rich repeats. J Biol Chem 268:26692-26698.
Higuchi, S., Nakamura, Y., and Saito, S. 2002. Characterization of a VNTR polymorphism in the coding region of the CEL gene. J Hum Genet 47:213-215.
Holtsberg, F.W., Ozgur, L.E., Garsetti, D.E., Myers, J., Egan, R.W., and Clark, M.A. 1995. Presence in human eosinophils of a lysophospholipase similar to that found in the pancreas. Biochem J 309 (Pt 1):141-144.
Horiuchi, S., Sakamoto, Y. and Sakai, M. 2003. Scavenger receptors for oxidized and glycated proteins. Amino Acids 25:283-91.
Howles, P.N., Carter, C.P., and Hui, D.Y. 1996. Dietary free and esterified cholesterol absorption in cholesterol esterase (bile salt-stimulated lipase) gene-targeted mice. J Biol Chem 271:7196-7202
Hui, D.Y. 1996. Molecular biology of enzymes involved with cholesterol ester hydrolysis in mammalian tissues. Biochim Biophys Acta 1303:1-14.
Hui, D.Y., and Howles, P.N. 2002. Carboxyl ester lipase: structure-function relationship and physiological role in lipoprotein metabolism and atherosclerosis. J Lipid Res 43:2017-2030.
Hurt-Camejo, E., Camejo, G., Rosengren, B., Lopez, F., Ahlstrom, C., Fager, G., and Bondjers, G. 1992. Effect of arterial proteoglycans and glycosaminoglycans on low density lipoprotein oxidation and its uptake by human macrophages and arterial smooth muscle cells. Arterioscler Thromb 12:569-583.
Kirby, R.J., Zheng, S., Tso, P., Howles, P.N., and Hui, D.Y. 2002. Bile salt-stimulated carboxyl ester lipase influences lipoprotein assembly and secretion in intestine: a process mediated via ceramide hydrolysis. J Biol Chem 277:4104-4109.
Li, F., and Hui, D.Y. 1997. Modified low density lipoprotein enhances the secretion of bile salt-stimulated cholesterol esterase by human monocyte-macrophages species- specific difference in macrophage cholesteryl ester hydrolase. J Biol Chem 272:28666-28671.
Li, F., and Hui, D.Y. 1998. Synthesis and secretion of the pancreatic-type carboxyl ester lipase by human endothelial cells. Biochem J 329:675-679.
Lidberg, U., Nilsson, J., Stromberg, K., Stenman, G., Sahlin, P., Enerback, S., and Bjursell, G. 1992. Genomic organization, sequence analysis, and chromosomal localization of the human carboxyl ester lipase (CEL) gene and a CEL-like (CELL) gene. Genomics 13:630-640.
Lidmer, A.S., Kannius, M., Lundberg, L., Bjursell, G., and Nilsson, J. 1995. Molecular cloning and characterization of the mouse carboxyl ester lipase gene and evidence for expression in the lactating mammary gland. Genomics 29:115-122.
Lindquist, S., Blackberg, L., and Hernell, O. 2002. Human bile salt-stimulated lipase has a high frequency of size variation due to a hypervariable region in exon 11. Eur J Biochem 269:759-767.
Lombardo. D., Guy, O. and Figarella, C. 1978. Purification and characterization of a carboxyl ester hydrolase from human pancreatic juice. Biochim Biophys Acta 527: 142-49.
Loomes, K.M., Senior, H.E., West, P.M., and Roberton, A.M. 1999. Functional protective role for mucin glycosylated repetitive domains. Eur J Biochem 266:105-111.
Madeyski, K., Lidberg, U., Bjursell, G., and Nilsson, J. 1999. Characterization of the gorilla carboxyl ester lipase locus, and the appearance of the carboxyl ester lipase pseudogene during primate evolution. Gene 239:273-282.
Moriwaki, H., Kume, N., Sawamura, T., Aoyama, T., Hoshikawa, H., Ochi, H., Nishi, E., Masaki, T., and Kita, T. 1998. Liga nd specificity of LOX-1, a novel endothelial receptor for oxidized low density lipoprotein. Arterioscler Thromb Vasc Biol 18:1541-1547.
Napoli, C., D'Armiento, F.P., Mancini, F.P., Postiglione, A., Witztum, J.L., Palumbo, G., and Palinski, W. 1997. Fatty streak formation occurs in human fetal aortas and is greatly enhanced by maternal hypercholesterolemia. Intimal accumulation of low density lipoprotein and its oxidation precede monocyte recruitment into early atherosclerotic lesions. J Clin Invest 100:2680-2690.
Navab, M., Berliner, J.A., Watson, A.D., Hama, S.Y., Territo, M.C., Lusis, A.J., Shih, D.M., Van Lenten, B.J., Frank, J.S., Demer, L.L., et al. 1996. The Yin and Yang of oxidation in the development of the fatty streak. A review based on the 1994 George Lyman Duff Memorial Lecture. Arterioscler Thromb Vasc Biol 16:831-842.
Nilsson, J., Blackberg, L., Carlsson, P., Enerback, S., Hernell, O., and Bjursell, G. 1990. cDNA cloning of human-milk bile-salt-stimulated lipase and evidence for its identity to pancreatic carboxylic ester hydrolase. Eur J Biochem 192:543-550.
Olofsson SO, Bostrom K, Svanberg U, Bondjers G. 1980. Isolation and partial characterization of a polypeptide belonging to apolipoprotein B from low-density lipoproteins of human plasma. Biochemistry. 19:1059-64.
Pentikainen, M.O., Oksjoki, R., Oorni, K., and Kovanen, P.T. 2002. Lipoprotein lipase in the arterial wall: linking LDL to the arterial extracellular matrix and much more. Arterioscler Thromb Vasc Biol 22:211-217.
Proctor, S.D., Vine, D.F., and Mamo, J.C. 2002. Arterial retention of apolipoprotein B(48)- and B(100)-containing lipoproteins in atherogenesis. Curr Opin Lipidol 13:461-470.
Quinn MT, Parthasarathy S, Steinberg D. 1988. Lysophosphatidylcholine: a chemotactic factor for human monocytes and its potential role in atherogenesis. Proc Natl Acad Sci U S A. 85:2805-9.
Reue, K., Zambaux, J., Wong, H., Lee, G., Leete, T.H., Ronk, M., Shively, J.E., Stern by, B., Borgstrom, B., Ameis, D., et al. 1991. cDNA cloning of carboxyl ester lipase from human pancreas reveals a unique proline-rich repeat unit. J Lipid Res 32:267-276.
Ross R. 1993. The pathogenesis of atherosclerosis: a perspective for the 1990s. Nature, 362:801-9.
Sakai M, Kobori S, Miyazaki A, Horiuchi S. 2000. Macrophage proliferation in atherosclerosis. Curr Opin Lipidol. 11:503-9.
Sawamura, T., Kume, N., Aoyama, T., Moriwaki, H., Hoshikawa, H., Aiba, Y., Tanaka, T., Miwa, S., Katsura, Y., Kita, T., et al. 1997. An endothelial receptor for oxidized low-density lipoprotein. Nature 386:73-77.
Schwenke, D.C., and Carew, T.E. 1989. Initiation of atherosclerotic lesions in cholesterol-fed rabbits. I. Focal increases in arterial LDL concentration precede development of fatty streak lesions. Arteriosclerosis 9:895-907.
Shamir, R., Johnson, W.J., Morlock-Fitzpatrick, K., Zolfaghari, R., Li, L., Mas, E., Lombardo, D., Morel, D.W., and Fisher, E.A. 1996. Pancreatic carboxyl ester lipase: a circulating enzyme that modifies normal and oxidized lipoproteins in vitro. J Clin Invest 97:1696-1704.
Skalen K, Gustafsson M, Rydberg EK, Hulten LM, Wiklund O, Innerarity TL, Boren J. 2002. Subendothelial retention of atherogenic lipoproteins in early atherosclerosis. Nature, 417:750-4.
Stromqvist, M., Hernell, O., Hansson, L., Lindgren, K., Skytt, A., Lundberg, L., Lidmer, A.S., and Blackberg, L. 1997. Naturally occurring variants of human milk bile salt-stimulated lipase. Arch Biochem Biophys 347:30-36.
Tulenko, T.N. and Sumner, A.E. 2002. The physiology of lipoproteins. J Nucl Cardiol 9:638-49.
Wang, C.S., and Hartsuck, J.A. 1993. Bile salt-activated lipase. A multiple function lipolytic enzyme. Biochim Biophys Acta 1166: 1-19.
Wang, C.S., and Johnson, K. 1983. Purification of human milk bile salt-activated lipase. Anal Biochem 133:457-461
Weng, W., Li, L., van Bennekum, A.M., Potter, S.H., Harrison, E.H., Blaner, W.S., Breslow, J.L., and Fisher, E.A. 1999. Intestinal absorption of dietary cholesteryl ester is decreased but retinyl ester absorption is normal in carboxyl ester lipase knockout mice. Biochemistry 38:4143-4149.
Williams, K.J., and Tabas, I. 1995. The response-to-retention hypothesis of early atherogenesis. Arterioscler Thromb Vasc Biol 15:551-561.
Winkler, K.E., Harrison, E.H., Marsh, J.B., Glick, J.M., and Ross, A.C. 1992. Characterization of a bile salt-dependent cholesteryl ester hydrolase activity secreted from HepG2 cells. Biochim Biophys Acta 1126:151-158.

## Claims

1. An *in vitro* method of identifying a compound useful for reducing the retention of atherogenic lipoproteins in atherogenesis which comprises assaying the compound for its ability to decrease the binding affinity of carboxyl ester lipase (CEL) to a receptor selected from vascular proteoglycans, scavenger receptors, AGE receptors, lipoprotein lipase, apolipoproteins, lipoproteins and lipoprotein particles.

2. The method of Claim 1 wherein the receptor is a scavenger receptor selected from SR-A types I, II and III, MARCO, SR-BI, CD36, SR-C1, SR-D, Macrosialin/CD86, SR-E, LOX-1 (lectin-like ox-LDL receptor), SR-F, SREC-1, SR-PSOX, FEEL-1, FEEL-2.

3. The method of Claim 1 wherein the receptor is an AGE receptor selected from RAGE, 80K-H, OST48, Galectin-3.

4. The method of Claim 1 wherein the receptor is an apolipoprotein selected from apo A-I, apo A-II, apo B-100, apo B-48, apo C-I, apo C-II, apo C-III, and apo E.

5. The method of Claim 1 wherein the receptor is a lipoprotein or lipoprotein particle selected from -
(a) the IDLs (intermediate-density lipoproteins) IDL1, IDL2 and IDL3,
(b) the LDLs (low density lipoproteins) LDL1, LDL2 and LDL3, and
(c) the HDLs (high-density lipoproteins) preβ-HDL, α-HDL, HDL1, HDL2, HDL3.

6. The method of Claim 1 which involves measurement of the ability of a test compound to decrease the binding affinity of CEL to the receptor using chromatographic methods with CEL or the receptor as the stationary phase.

7. The method of Claim 1 which involves measurement of the ability of a test compound to decrease the binding affinity of CEL to cells expressing the receptor on the cellular surface, preferably using labelled CEL.

8. The method of Claim 1 which involves measurement of the ability of a test compound to decrease the binding affinity of CEL to the receptor using scintillation proximity and ultracentrifugation, preferably using labelled CEL.

9. The method of Claim 1 which involves measurement of the ability of a test compound to decrease the binding affinity of CEL to vascular tissue, preferably using labelled CEL.

## Patentansprüche

1. *In vitro*-Verfahren zum Identifizieren einer Verbindung, die zum Reduzieren der Retention von atherogenen Lipoproteinen bei Atherogenese verwendbar ist, wobei das Verfahren das Testen der Verbindung auf ihre Fähigkeit aufweist, die Bindungsaffinität von Carboxylesterlipase (CEL) an einen Rezeptor zu reduzieren, der aus vaskulären Proteoglykanen, Scavenger-Rezeptoren, AGE-Rezeptoren, Lipoproteinlipase, Apolipoproteinen, Lipoproteinen und Lipoproteinpartikeln ausgewählt ist.

2. Verfahren nach Anspruch 1, wobei der Rezeptor ein Scavenger-Rezeptor ist, der aus SR-A-Typ I, II oder III, MARCO, SR-B1, CD36, SR-C1, SR-D, Macrosialin/CD86, SR-E, LOX-1 (Lectin-artiger Ox-LDL-Rezeptor), SR-F, SREC-1, SR-PSOX, FEEL-1, FEEL-2 ausgewählt ist.

3. Verfahren nach Anspruch 1, wobei der Rezeptor ein AGE-Rezeptor ist, der aus RAGE, 80K-H, OST48, Galectin-3 ausgewählt ist.

4. Verfahren nach Anspruch 1, wobei der Rezeptor ein Apolipoprotein ist, das aus Apo A-I, Apo A-II, Apo B-100, Apo B-48, Apo C-I, Apo C-II, Apo C-III und Apo E ausgewählt ist.

5. Verfahren nach Anspruch 1, wobei der Rezeptor ein Lipoprotein oder ein Lipoproteinpartikel ist, das/der ausgewählt ist aus:
(a) den IDLs (Intermediate-Density Lipoproteins = Lipoproteinen mittlerer Dichte) IDL1, IDL2 und IDL3,
(b) den LDLs (Low-Density Lipoproteins = Lipoproteinen geringer Dichte) LDL1, LDL2 und LDL3, und
(c) den HDLs (High-Density Lipoproteins = Lipoproteinen hoher Dichte) preβ-HDL, α-HDL, HDL1, HDL2, HDL3.

6. Verfahren nach Anspruch 1, das das Messen der Fähigkeit einer Testverbindung, die Bindungsaffinität von CEL an einen Rezeptor zu reduzieren, unter Verwendung von chromatographischen Verfahren mit CEL oder dem Rezeptor als stationäre Phase einbezieht.

7. Verfahren nach Anspruch 1, das das Messen der Fähigkeit einer Testverbindung, die Bindungsaffinität von CEL an Zellen, die den Rezeptor an einer Zelloberfläche exprimieren, zu reduzieren, vorzugsweise unter Verwendung von markierter CEL, einbezieht.

8. Verfahren nach Anspruch 1, das das Messen der Fähigkeit einer Testverbindung, die Bindungsaffinität von CEL an einen Rezeptor zu reduzieren, unter Verwendung von Scintillation Proximity und Ultrazentrifugation, vorzugsweise unter Verwendung markierter CEL, einbezieht.

9. Verfahren nach Anspruch 1, das das Messen der Fähigkeit einer Testverbindung, die Bindungsaffinität von CEL an vaskuläres Gewebe zu reduzieren, vorzugsweise unter Verwendung markierter CEL, einbezieht.

## Revendications

1. Procédé *in vitro* pour identifier un composé utile pour réduire la rétention de lipoprotéines athérogènes dans l'athérogenèse, qui consiste à tester le composé pour son aptitude à réduire l'affinité de liaison de la carboxyl ester lipase (CEL) à un récepteur sélectionné parmi les protéoglycanes vasculaires, les récepteurs « poubelle », les récepteurs des AGE, la lipoprotéine lipase, les apolipoprotéines, les lipoprotéines et les particules lipoprotéiques.

2. Procédé selon la revendication 1, dans lequel le récepteur est un récepteur « poubelle » sélectionné parmi SR-A types I, II et III, MARCO, SR-BI, CD36, SR-C1, SR-D, macrosialine/CD86, SR-E, LOX-1 (récepteur des LDL oxydées de type lectine), SR-F, SREC-1, SR-PSOX, FEEL-1, FEEL-2.

3. Procédé selon la revendication 1, dans lequel le récepteur est un récepteur des AGE sélectionné parmi RAGE, 80K-H, OST48, Galectine-3.

4. Procédé selon la revendication 1, dans lequel le récepteur est une apolipoprotéine sélectionnée parmi apo A-I, apo A-II, apo B-100, apo B-48, apo C-I, apo C-II, apo C-III, et apo E.

5. Procédé selon la revendication 1, dans lequel le récepteur est une lipoprotéine ou une particule lipoprotéique sélectionnée parmi
(a) les IDL (lipoprotéines de densité intermédiaire) IDL1, IDL2 et IDL3,
(b) les LDL (lipoprotéines de faible densité) LDL1, LDL2 et LDL3, et
(c) les HDL (lipoprotéines de haute densité) préβ-HDL, α-HDL, HDL1, HDL2, HDL3.

6. Procédé selon la revendication 1, qui implique de mesurer l'aptitude d'un composé de test à réduire l'affinité de liaison de la CEL au récepteur en utilisant des procédés chromatographiques avec la CEL ou le récepteur en tant que phase stationnaire.

7. Procédé selon la revendication 1, qui implique de mesurer l'aptitude d'un composé de test à réduire l'affinité de liaison de la CEL à des cellules exprimant le récepteur sur la surface cellulaire, en utilisant de préférence une CEL marquée.

8. Procédé selon la revendication 1, qui implique de mesurer l'aptitude d'un composé de test à réduire l'affinité de liaison de la CEL au récepteur en utilisant une scintillation de proximité et une ultracentrifugation, en utilisant de préférence une CEL marquée.

9. Procédé selon la revendication 1, qui implique de mesurer l'aptitude d'un composé de test à réduire l'affinité de liaison de la CEL à un tissu vasculaire, en utilisant de préférence une CEL marquée.
